# EUROPEAN PATENT APPLICATION

(11) **EP 1 656 934 A1**
(43) Date of publication of application: **17.05.2006**
(21) Application number: 04026981.3
(22) Date of filing: 12.11.2004
(51) Int. Cl.: A61K 31/20, A61K 31/201, A61K 31/202, A61K 31/23, A61K 31/231, A61K 31/232, A61K 31/575, A61K 31/704, A61P 3/00

(54) **Use of physiologically active fatty acids for treating lipodystrophy**

(71) Applicant: Cognis IP Management GmbH, 40589 Düsseldorf (DE)
(72) Inventor: Prous, Santiago Rull, 08034 Barcelona (ES)

(57) **Abstract**

Disclosed is the use of physiologically active fatty acids, their salts and their esters, optionally in combination with extracts of *Castanea sativa* or its active principles for making a medicament for fighting lipodystrophy.

## Description

### Object of the invention

The present invention relates to the area of pharmaceutical compositions and provides the use of a new active agent or composition for fighting lipodystrophy.

### State of the art

Lipodystrophy means a disorder of adipose (fatty) tissue characterized by a selective loss of body fat. Typical symptoms of this disease are
- sunken cheeks,
- fat increase in the face,
- prominent veins in the legs,
- fat loss in the legs and arms,
- loss of shape in the buttocks,
- fat increase around the stomach (called truncal or central obesity),
- enlarged breasts,
- fat pad in the neck (sometime called buffalo hump), and
- lipomas (fatty growth in different parts of the body).

Patients suffering from lipodystrophy have a tendency to develop insulin resistance, diabetes, a high triglyceride and cholesterol level (hypertriglyceridemia) in serum, and fatty liver. There are numerous forms of lipodystrophy that are genetic (inherited) or acquired (not inherited). The genetic forms of lipodystrophy include congenital generalized lipodystrophy (the Berardinelli-Seip syndrome) and several types of familial partial lipodystrophy (the Dunnigan type, the Köbberling type, the mandibuloacral dysplasia type). The acquired forms of lipodystrophy include acquired generalized lipodystrophy (the Lawrence syndrome), acquired partial lipodystrophy (the Barraquer-Simons syndrome), and lipodystrophy induced by protease inhibitors used to treat HIV.

From the state of the art there are known, for example, adipose-targeting peptides **[WO 03/022991 A1**, University of Texas] or anti-estrogens like clomiphen **US 20040171697 A1** (Zonagen)] for fighting lipodystrophy. According to **US6365176 B1** (Functional Food) it is suggested to add a nutritional supplement to food, comprising a low-glycemic index carbohydrate source, a source of protein, a source of fat, a source of sterol and/or stanol, a source of chromium, a source of salicylic acid, and a source of ginseng in order to reduce triglyceride and cholesterol level in blood. Nevertheless, up to now none of the actives or compositions have been found to give satisfying results. Therefore, the underlying problem of the present invention has been to develop a new medicament for fighting lipodystrophy, which exhibits a better performance with respect to fat distribution in the human body and to dermatological and toxicological safety.

### Description of the invention

A first object of the present invention claims the use of physiologically active fatty acids, their salts and their esters for preparing a medicament for fighting lipodystrophy.

A second object of the present invention is directed at active compositions comprising
(a) physiologically active fatty acids, their salts and their esters, and
(b) extracts of *Castanea sativa* or its active principles
for preparing a medicament for fighting lipodystrophy.

Surprisingly, it has been found that physiologically active fatty acids, predominantly unsaturated fatty acids like conjugated linoleic acid (CLA) or omega-3 fatty acids, which are mainly derived from marine sources, their salts and esters improves the fat distribution in the human body and fights the disorder of adipose tissue, which is a main cause for lipodystrophy. In combination with extracts of *Castanea sativa* in general, or its main active ingredient, β-escin, synergistic effects have been found. In addition, while the fatty acids administered alone need some time to develop the benefits, the mixtures exhibit a higher activity. Finally, both, the fatty acids as well as the mixtures are toxicologically and dermatologically safe.

### Physiologically active fatty acids, their salts and their esters

A common criterion for fatty acids with physiological activity, which represent component (a), is a fat chain having a sufficient number of carbon atoms providing a lipophilic behaviour that allows the molecule to pass through the gastrointestinal tract of the body, and a sufficient number of double bonds. Therefore, said fatty acids usually comprise 18 to 26 carbon atoms and 2 to 6 double bonds.

In a first embodiment of the present invention, conjugated linoleic acid (CLA) or its alkaline or alkaline earth salts and esters, preferably esters with lower aliphatic alcohols having 1 to 4 carbon atoms ― or their glycerides, specially their triglycerides, come into account. Conjugated linoleic acid (CLA) represents a commercially available product which usually is obtained by base-catalysed isomerisation of sunflower oil or its respective alkyl esters and subsequent isomerisation in the presence of enzymes. CLA is an acronym used for positional and geometric isomers deriving from the essential fatty acid linoleic acid (LA, cis-9,cis-12-octadecadienoic acid, 18:2n-6). From a physiological point of view, the use of the *cis-*9*,trans-*11 isomer, according to the present invention, is of special importance having at least 30, preferably at least 50 and most preferably at least 80 % b.w. of said *cis-*9*,trans-*11 isomer, based on the total CLA content of the crude mixture. In addition, it has been found advantageous if the content of the *trans-*10*,cis-*12 isomer is at most 45, preferably at most 10 % b.w. and most preferably is less than 1 % b.w., and the sum of 8,10-, 11,13- and *trans,trans*isomers in total is less than 1 % b.w. - again based on the total CLA content. Such products can be found in the market, for example, under the trademark Tonalin® CLA-80 (Cognis).

In a second embodiment, also so-called omega-3 fatty acids can come into account, which typically comprise 18 to 26, preferably 20 to 22 carbon atoms and at least 4 and up to 6 double bonds. Also these molecules are very well known from the art and can be obtained by standard methods of organic chemistry, for example, via transesterification of fish oils, followed by urea precipitation of the alkyl esters thus obtained, and a final extraction using non-polar solvents as described in the German patent **DE 3926658 C2** (Norsk Hydro). Fatty acids thus obtained are rich in omega-3 (all-Z)-5,8,11,14,17-eicosapentanoic acid (EPA) C 20 : 5 and (all-Z)-4,7,10,13,16,19-docosahexanoic acid (DHA) C 22 : 6. Such products can be found in the market under the trademark Omacor® (Pronova).

In a third embodiment, also linoleic acid, vaccinic acid (trans 11-octadecenoic acid) or cishexadecenoic acid (obtained for example from the plant *Thunbergia alata)* can be used.

In addition, said physiologically active fatty acid esters can not only be used in form of their lower alkyl esters or glycerides, an additional well-preferred embodiment of the present invention relates to compositions comprising esters of said fatty acids with sterols. Like glycerides, sterol esters are easily resorbed and splitted by the human body, however, a significant advantage comes from the fact that the cleavage of the ester bond releases a second molecule with health promoting properties. To avoid unclarities, the phrases "sterol", "stanol" and "sterin" shall be used as synonyms defining steroids showing a single hydroxyl group linked to the C-3. In addition sterols, which consist of 27 to 30 carbon atoms, may show a double bond, preferably in 5/6 position. According to the present invention, esters of CLA or omega-3 fatty acids with β-sitosterol or its hydrogenation product β-sitostanol are preferred.

### Extracts of Castanea sativa and its active principles

Main ingredients of the extracts of horse chestnuts *(Castanea sativa),* which represent component (b), are saponins and escin, which is a mixture of two glycosides whose aglycons are derived from proteoescigenin, while the sugars represent either glucuronic acid of two molecules D-glucose. Said glycosides differ in the acyl groups in the C22-position.

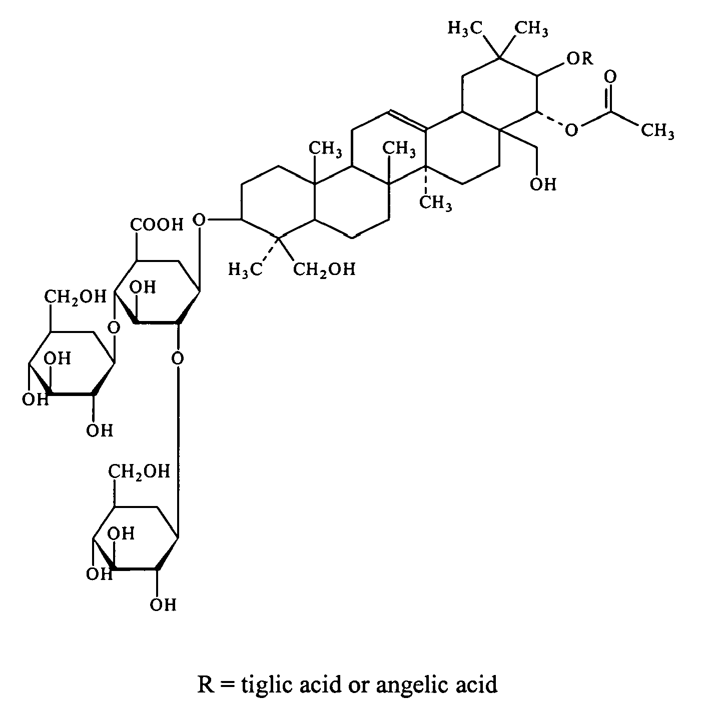

While α-escin represents an amorphous powder, which melts at between 225 and 227 °C and is easily soluble in water, β-escin (which is also called flogencyl) forms flakes which are practically water-insoluble, but can be dissolved in alcohol.

### Active compositions

The compositions according to the present invention - either component (a) alone or mixtures of components (a) and (b) - may be administered either topically or orally. Mixtures can comprise component (a) and component (b) in a weight ratio of from 99 : 1 to 50 : 50 and more particularly from 95 : 10 to 75 : 25. The highest synergistic effects, however, are observed at ratios from 92 : 8 to 80 : 20. In general, the compositions can be used in a concentration of up to about 10, particularly 0.5 to 8 and more particularly 1 to 2 % b.w. ― calculated the final composition. One percent, however, has been found to be particularly suitable.

In order to support the anti-lipodystrophic effect by fighting cellulite and lightening skin, the compositions may comprise further plant extracts or their active principles, for example, chosen from the plants selected from the group consisting of *Ginkgo biloba, Oleacea europensis, Glyzyrrhiza glabra, Vaccinium myrtillus, Trifolium pratense, Litchi sinensis, Vitis, vinifera, Brassica oleracea, Punica granatum, Petroselinium crispum, Centella asiatica, Passiflora incarnata, Medicago sativa, Valeriana officinalis, Salix alba,* and *Harpagophytum procumbens.*

### Macro- or micro-encapsulation

In a special embodiment of the present invention, said compositions are macro- or micro-encapsulated. "Microcapsules" are understood to be spherical aggregates with a diameter from about 0.1 to about 5 mm which contain at least one solid or liquid core surrounded by at least one continuous membrane. More precisely, they are finely dispersed liquid or solid phases coated with film-forming polymers, in the production of which the polymers are deposited onto the material to be encapsulated after emulsification and coacervation or interfacial polymerization. In another process, liquid active principles are absorbed in a matrix ("micro-sponge") and, as microparticles, may be additionally coated with film-forming polymers. The microscopically small capsules, also known as nanocapsules, can be dried in the same way as powders. Besides single-core microcapsules, there are also multiple-core aggregates, also known as microspheres, which contain two or more cores distributed in the continuous membrane material. In addition, single-core or multiple-core microcapsules may be surrounded by an additional second, third, etc., membrane. The membrane may consist of natural, semisynthetic or synthetic materials. Natural membrane materials are, for example, gum arabic, agar agar, agarose, maltodextrins, alginic acid and salts thereof, for example sodium or calcium alginate, fats and fatty acids, cetyl alcohol, collagen, chitosan, lecithins, gelatin, albumin, shellac, polysaccharides, such as starch or dextran, polypeptides, protein hydrolysates, sucrose, and waxes. Semisynthetic membrane materials are, inter alia, chemically modified celluloses, more particularly cellulose esters and ethers, for example, cellulose acetate, ethyl cellulose, hydroxypropyl cellulose, hydroxypropyl methyl cellulose, and carboxymethyl cellulose, and starch derivatives, more particularly starch ethers and esters. Synthetic membrane materials are, for example, polymers, such as polyacrylates, polyamides, polyvinyl alcohol or polyvinyl pyrrolidone. Examples of known microcapsules are the following commercial products (the membrane material is shown in brackets) *Hallcrest Microcapsules* (gelatin, gum arabic), *Coletica Thalaspheres* (maritime collagen), *Lipotec Millicapseln* (alginic acid, agar agar), *Induchem Unispheres* (lactose, microcrystalline cellulose, hydroxypropylmethyl cellulose), *Unicerin C30* (lactose, microcrystalline cellulose, hydroxypropylmethyl cellulose), *Kobo Glycospheres* (modified starch, fatty acid esters, phospholipids), *Softspheres* (modified agar agar), *Kuhs Probiol Nanospheres* (phospholipids), and *Primaspheres* or *Primasponges* (chitosan, anionic polymers). The encapsulation of the compositions according to the present invention is preferred in case the active should be liberated under controlled release. Therefore, a person skilled in the art can easily select the adequate encapsulation system by comparing the stability of the capsules under the pH-conditions of the respective part of the intestine. Nevertheless, a very well working procedure has been found to use a combination of proteins like gelatin and anionic polymers, e.g., carboxy methyl cellulose. For example, a first aqueous solution of gelatin and a second aqueous solution of CMC and the active ingredients are mixed under vigorous stirring at a temperature above the melting point of the protein. Once the mixture has been allowed to cool down, the coacervation of the micro-capsules takes place indicated by an increasing turbidity of the solution. For hardening of the capsules it is suggested to reticulate the shells by adding a cross-linking agent, for example, glutaraldehyde. A detailed description of the process is given, for example, in **EP 0937496 A2** (Unilever). Other suitable encapsulation methods based on gel formation agents, anionic polymers and chitosan have been described, for example, in **WO 01/01926, WO 01/01927, WO 01/01928, WO 01/01929** (Primacare), all of them being incorporated by reference.

### Examples

### Example 1

Two solutions, one containing 2.8 % b.w. gelatin (Geltec® SG-730 N, Extraco) and one containing 1.2 % b.w. sodium carboxymethylcellulose (DS-07, Aldrich, MW = 90.000) and 1.5 % b.w. conjugated linoleic acid (Tonalin® CLA, Cognis) were prepared in distilled water at 45 °C. Both solutions were adjusted to pH 6.5 using a 1 N NaOH solution. The CMC solution was added to the solution containing the gelatin, and dispersed using Ultra Turrax equipment. Thereafter, the pH was adjusted to 4.25 using a 1 N HCl solution and the mixture was cooled down to 25 °C under stirring. Subsequently, the formation of coacervates was recognized by increasing turbidity. The so-formed coacervates were hardened by adding glutaraldehyde under stirring. After a reaction time of 4 h at 25 °C, the coacervates thus obtained were filtered off using a Buchner funnel and stored at 5 °C as a slurry. The average particle size was 12.8 µm.

### Example 2

Two solutions, one containing 2.5 % b.w. gelatin (Geltec® SG-730 N, Extraco) and one containing 1.3 % b.w. sodium carboxymethylcellulose (AV 27088D, Aldrich, MW = ca. 38.000), 1.2 % b.w. conjugated linoleic acid (Tonalin® CLA, Cognis) and 0.5 % b.w. of a spray-dried extract of *Castanea sativa* (Herbalia® Horse chestnut, Cognis) were prepared in distilled water at 45 °C. Both solutions were adjusted to pH 6.5 using a 1 N NaOH solution. The CMC solution was added to the solution containing the gelatin and dispersed using Ultra Turrax equipment. Thereafter, the pH was adjusted to 4.25 using a 1 N HCl solution and the mixture was cooled down to 25 °C under stirring. Subsequently, the formation of coacervates was recognized by increasing turbidity. The so-formed coacervates were hardened by adding glutaraldehyde under stirring. After a reaction time of 4 h at 25 °C, the coacervates thus obtained were filtered off using a Buchner funnel and stored at 5 °C as a slurry. The average particle size was 45.5 µm.

### Example 3

Two solutions, one containing 2.8 % b.w. gelatin (Geltec® SG-730 N, Extraco) and one containing 1.2 % b.w. sodium carboxymethylcellulose (DS-07, Aldrich, MW = 90.000) and 1.5 % b.w. ω-unsaturated fish fatty acid (Omacor® , Pronova) were prepared in distilled water at 45 °C. Both solutions were adjusted to pH 6.5 using a 1 N NaOH solution. The CMC solution was added to the solution containing the gelatin and dispersed using Ultra Turrax equipment. Thereafter, the pH was adjusted to 4.25 using a 1 N HCl solution, and the mixture was cooled down to 25 °C under stirring. The so-formed coacervates were hardened by adding glutaraldehyde under stirring. After a reaction time of 4 h at 25 °C the coacervates thus obtained were filtered off using a Buchner funnel and stored as a slurry at 5 °C. The average particle size was 12.5 µm.

### Example 4

Two solutions, one containing 2.8 % b.w. gelatin (Geltec® SG-730 N, Extraco) and one containing 1.2 % b.w. sodium carboxymethylcellulose (DS-07, Aldrich, MW = 90.000) and 1.5 % b.w. of a synthetic triglyceride based on conjugated linoleic acid (Tonalin CLA-TG® , Cognis), were prepared in distilled water at 45 °C. Both solutions were adjusted to pH 6.5 using a 1 N NaOH solution. The CMC solution was added to the solution containing the gelatin and dispersed using Ultra Turrax equipment. Thereafter, the pH was adjusted to 4.25 using a 1 N HCl solution and the mixture was cooled down to 25 °C under stirring. After a reaction time of 4 h at 25 °C, the coacervates thus obtained were filtered off using a Buchner funnel and stored at 5 °C as a slurry. The average particle size was 12.5 µm.

### Example 5

Two solutions, one containing 2.8 % b.w. gelatin (Geltec® SG-730 N, Extraco) and one containing 1.2 % b.w. sodium carboxymethylcellulose (DS-07, Aldrich, MW = 90.000) and 1.5 % b.w. conjugated linoleic acid sterol ester were prepared in distilled water at 45 °C. Both solutions were adjusted to pH 6.5 using a 1 N NaOH solution. The CMC solution was added to the solution containing the gelatin and dispersed using Ultra Turrax equipment. Thereafter, the pH was adjusted to 4.25 using a 1 N HCl solution, and the mixture was cooled down to 25 °C under stirring. After a reaction time of 4 h at 25 °C, the coacervates thus obtained were filtered off using a Buchner funnel and stored at 5 °C as a slurry. The average particle size was 12.5 µm.

## Claims

1. Use of physiologically active fatty acids, their salts and their esters for making a medicament for fighting lipodystrophy.

2. Use of active compositions comprising
(a) physiologically active fatty acids, their salts and their esters, and
(b) extracts of *Castanea sativa* or its active principles
for making a medicament for fighting lipodystrophy.

3. Use according to claims 1 or 2, **characterised in that** said physiologically active fatty acids (component a) comprise from 18 to 26 carbon atoms and from 2 to 6 double bonds.

4. Use according to any of the claims 1 to 3, **characterised in that** component (a) represents esters of said fatty acids with glycerol or sterol.

5. Use according to any of the claims 1 to 4, **characterised in that** component (a) represents conjugated linoleic acid or omega-3 fatty acids.

6. Use according to any of the claims 2 to 5, **characterised in that** component (b) represents β-escin or an extract of *Castanea sativa* rich in β-escin.

7. Use according to any of the claims 2 to 6, **characterised in that** they comprise component (a) and (b) in weight ratios of from 99 : 1 to 50 : 50.

8. Use according to any of the claims 1 to 7, **characterised in that** said medicaments are macro- or micro-encapsulated.
